Europäisches Patentamt

European Patent Office

Office européen des brevets

(11) Publication number: **0 204 922**

**A2**

(12) # EUROPEAN PATENT APPLICATION

(21) Application number: **86105151.4**

(22) Date of filing: **15.04.86**

(51) Int. Cl.⁴: **G 01 N 33/574**
**//G01N33/554**

(30) Priority: **28.05.85 US 737986**

(43) Date of publication of application:
**17.12.86 Bulletin 86/51**

(84) Designated Contracting States:
**BE DE FR IT**

(71) Applicant: **ABBOTT LABORATORIES**
**14th Street and Sheridan Road North St**
**North Chicago Illinois 60064(US)**

(72) Inventor: **Miller, Larry S.**
**2632 Vermont Avenue**
**Waukegan Illinois 60087(US)**

(72) Inventor: **Doyle, David E.**
**1222 South Ridge Avenue**
**Arlington Heights Illinois 60005(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) Immunohistochemical assay for detection of a tumor-associated marker (p53).

(57) This invention is an immunohistochemical p53 assay method in which human tissue sections or cells are fixed on a glass slide and incubated with anti-p53 antibodies which react with the p53 antigen in the specimen. The p53 antigen-antibody complex is then localized by addition of anit-Ig which is either labeled directly or indirectly with an enzyme. Next, a chromogen substrate solution for the enzyme is added to the slide and color is developed. The slide is then examined under a microscope for specifically stained cells which indicate the presence of p53 antigen.

EP 0 204 922 A2

## IMMUNOHISTOCHEMICAL ASSAY FOR DETECTION OF A
## TUMOR-ASSOCIATED MARKER (p53)

### Field of the Invention

The invention relates to a method for detecting a tumor-associated marker and more particularly to an immunohistochemical method for this purpose.

### Background of the Invention

A tumor-associated marker or antigen designated p53 was initially identified in animal tumors by Lane et al., Nature 278:261-263 (1979); De Leo et al., Proc. Natl. Acad. Sci. 76:2420-2424 (1979). The p53 molecule is a phosphoprotein having a molecular weight of 53,000 daltons which is usually found in the nucleus of tumor cells. Rotter et al., Int. J. Cancer 31:315-320 (1983), discovered that tumors which are positive for p53 tend to progress, whereas, tumors which are negative for p53 tend to regress. Also, the presence of p53 in the nucleus of cells may be indicative of transformation of normal cells to a tumorigenic state.

Crawford et al., Int. J. Cancer 30:403-408 (1982) and Benchimol et al., EMBO J. 1:1055-1062 (1982) have identified the presence of antibody to p53 in the sera of breast cancer patients using a solid phase radioimmunoassay and an immunoprecipitation assay. Further, Crawford et al. suggest that the presence of p53 antibody in breast cancer sera may be indicative of metastatic disease.

The detection of p53 in various tumor cell cultures has been performed using various antibodies specific for this antigen; see, for example, Lane et al., Nature 278:261-263 (1979); De Leo, et al., Proc. Natl. Acad. Sci. 76:2420-2424 (1979). Monoclonal antibodies have also been prepared to p53 from human and mouse cell cultures; see, for example, Thomas et al., Virology 131:502-517 (1983); Gurney, et al., J. Virology 34:752-763 (1980).

These prior art methods have utilized immunofluorescence for detection of p53 in animal cell lines and immunoprecipitation methods for detection of p53 in human cell lines. However, to our knowledge, p53 has not been localized by immunohistochemical procedures in human tumor specimens or human tumor cell lines.

## Summary of the Invention

We have developed an immunohistochemical p53 assay method which permits not only the detection of p53 antigen in human cells but also in tissue specimens. The improved p53 assay utilizes an immunoenzymatic staining method which is more sensitive and stable than prior art immunofluorescence assays and simpler to perform than immunoprecipitation methods.

According to the invention, human tissue sections or cells are fixed on a glass slide and incubated with anti-p53 antibodies which react with the p53 antigen in the specimen. The p53 antigen-antibody complex is then localized by addition of anti-Ig which is either labeled directly or indirectly with an enzyme. Next, a chromogen substrate solution for the enzyme is added to the slide and color is developed. The slide is then examined under a microscope for specifically stained cells which indicate the presence of p53 antigen.

## Detailed Description of the Invention

The method for detecting p53 in tumor cells or tissue according to this invention comprises fixing a tissue or cell preparation to be examined on a glass slide, contacting the fixed tissue or cells with antibody to p53, contacting the tissue or cells with anti-Ig labeled either directly or indirectly with an enzyme, contacting the tissue or cells with a chromogenic indicator which changes color in the presence of the enzyme and examining the slide for presence of p53.

For purposes of this disclosure, "anti-Ig labeled directly with an enzyme" means that an antibody to Ig is conjugated to an enzyme such as by the methods described by Nakane et al., *J. Histochem. Cytochem.* 22:1084-1091 (1974). The anti-Ig-enzyme conjugate comprises one reagent. "Anti-Ig labeled indirectly with an enzyme" means the anti-Ig is added to the assay as one reagent, and then a separate Ig-enzyme reagent is added which will bind to the anti-Ig in the inventive assay. An anti-Ig indirectly labeled with an enzyme is described in Example 1 below.

The following examples are illustrative of the inventive p53 assay.

### Example 1

This example illustrates a preferred procedure for conducting the assay method of this invention.

1. Treat microscope slides with a positively-charged polymer solution such as poly-l-lysine (molecular weight = 30,000-70,000) at 100 micrograms per milliliter in distilled water for 5 minutes and then rinse and dry. Other positively-charged polymer solutions such as poly-l-arginine can be used to pretreat the slide.

2. Cut a frozen section (6-8 microns) of tissue to be tested using a cryostat and transfer to poly-l-lysine coated slide. Any human cell or tissue preparation can be substituted for the frozen section.

3. Immediately fix the frozen tissue section on the slide in a fixative such as Zamboni's fixative at 4°C for 10-15 minutes, rinse in 0.01 M phosphate buffered saline at pH 7.4 (PBS) for 5 minutes, place in cold acetone at -20°C for 1 minute and finally rinse twice in phosphate buffered saline baths for 5 minutes each.

Steps 4-11 below are preferably performed at ambient temperatures of 18-25°C.

4. Add 2% or greater normal serum in phosphate buffered saline solution to tissue section and incubate 15 minutes. Remove excess reagent from the slide.

5. Add anti-p53 monoclonal antibody (preferably diluted 1:10 in 2% normal serum-phosphate buffered saline) to the tissue section and incubate 30 minutes. In the preferred embodiment, the anti-p53 monoclonal antibody is of mouse origin. Rinse twice in phosphate buffered saline baths for 5 minutes each.

6. Add anti-Ig antibody (preferably diluted 1:20 in phosphate buffered saline solution) to the tissue section and incubate 30 minutes. In the preferred assay, the anti-Ig antibody is species-specific for the species of origin for the anti-p53 antibody, such as an anti-mouse IgG antibody. Rinse twice in phosphate buffered saline baths for 5 minutes each.

7. Add peroxidase, anti-peroxidase (PAP) complex (preferably diluted 1:50 in 2% normal serum-phosphate buffered saline) to the tissue section and incubate 30 minutes. Again, in the preferred assay, the PAP complex is of the same species origin as the anti-p53 antibody such as mouse PAP complex. Rinse twice in phosphate buffered saline baths for 5 minutes each.

8. Prepare a chromogen substrate solution. Examples of chromogen substrate solutions are well known in the art and include diaminobenzidine, aminoethyl carbozole and 4-chloro-alpha-naphthol. In the preferred assay, 10 milligrams diaminobenzidine + 20 microliters of 30% hydrogen peroxide + 16 milliliters phosphate buffered saline is used as the chromogen solution. Add to tissue section and allow to incubate 6 minutes at ambient temperature. Rinse for 5 minutes in running tap water.

9. Counterstain section in diluted hematoxylin, malachite green or methylene blue. Preferably, Harris hematoxylin (1:10 in distilled water) is applied to the section for 10-15 seconds. Rinse for 5 minutes in running tap water.

10. Dehydrate section as, for example, by immersing the section twice for 2 minutes in 95% ethanol, twice for 2 minutes in absolute ethanol and twice for 2 minutes in xylene.

11. Examine the slide microscopically at, for example, 25 x 10 magnification power to detect specifically stained cells.

## Example 2

This example demonstrates the preparation of monoclonal antibody to p53 as described by Gurney et al., *J. Virology* 34:752-763 (1980).

1.  Balb/c mice are injected with SV40 transformed cells.

2.  Spleen cells from immunized animals are fused with a murine myeloma, NS1 according to the procedure described by Galfre et al., *Nature* 266:550-552 (1977), using polyethylene glycol 1000.

3.  The fused cells are grown in Dulbecco's minimum essential medium containing 20% fetal calf serum, 50 uM hypoxanthine, 5 uM aminopterin, and 10 uM thymidine in a multiwell plate.

4.  Culture supernatants from the wells showing growth are evaluated for antibodies to the p53 antigen by an enzyme-linked immunosorbent assay described by Engvall and Perlmann, *J. Immunology* 109:129-135 (1972).

5.  Positive cells are cloned in soft agar and expanded in larger culture dishes. The culture supernatants are collected and stored at -20°C.

## Example 3

The preparation of mouse peroxidase, anti-peroxidase complex (PAP) is based on the procedure of Sternberger et al., *J. Histochem. Cytochem.* 18:315-333 (1970).

1. Mice are injected with horseradish peroxidase (HRPO) to prepare antisera.

2. Mouse anti-HRPO serum is incubated with HRPO at room temperature.

3. The resulting precipitate is centrifuged at 12,000 g for 20 minutes at 4°C and washed three times with cold phosphate-buffered saline (PBS).

4. The precipitate is resuspended in a solution of HRPO and then dissolved by adjusting to pH 2.3 using 1N hydrochloric acid.

5. The solution is immediately neutralized to pH 7.4 using 1N sodium hydroxide.

6. Next, 2.7 ml of 0.08 N sodium acetate and 0.15 N ammonium acetate are added.

7. The precipitate is washed once in cold, 50% saturated ammonium sulfate.

8. The precipitate is dissolved in distilled water and dialyzed against sodium-ammonium acetate saline.

9. The dialyzed solution is centrifuged at 20,000 g for 16 minutes at 4°C to remove any remaining precipitate.

10. The resulting mouse PAP complex is aliquoted and stored at -20°C.

## Example 4

This example illustrates a procedure for the conjugation of an enzyme to anti-Ig as described by

Nakane et al., <u>J. Histochem. Cytochem.</u> 22:1084-1091 (1974).

1.  The enzyme, horseradish peroxidase (HRPO), is activated by treating with sodium periodate in 0.1M sodium bicarbonate, pH 8.0 for 30-35 minutes.

2.  The excess sodium periodate is removed from the HRPO by column chromatography such as on a Sephadex G-25 column, made by Pharmacia Inc., Piscataway, New Jersey, which has been equilibrated in 0.01M sodium carbonate, pH 9.5.

3.  The activated HRPO is added to anti-Ig and incubated for 2 hours at room temperature.

4.  The solution is cooled to 4°C in an ice bath.

5.  Sodium borohydride is dissolved in cold 0.01M sodium carbonate, pH 9.5 and added to the anti-Ig-HRPO conjugate for 12-24 hours at 4°C.

6.  Acetone is added to the solution to destroy excess sodium borohydride and allowed to stand for 30 minutes at room temperature.

7.  The anti-Ig-HRPO conjugate is diluted in a protein stabilizing solution and stored at -20°C.

There are many advantages to the above-described immunohistochemical assay for p53. First, the assay can be performed routinely in most pathology laboratories using standard frozen sections. Previous assays such as polyacrylamide gel electrophoresis and immunoprecipitation are not routinely performed in clinical laboratories. Second, the assay provides an immunoperoxidase staining technique which utilizes a highly stable reaction product. Therefore, stained specimens can be stored for several years without loss of localization of the p53 antigen. Immunofluorescent stained specimens, on the other hand, must be stored under special conditions in order to preserve localization. Third, the assay is easy to perform without any special instruction, training or equipment.

Although this invention has been described with respect to specific modifications, the details thereof are not to be construed as limitations, for it will be apparent that various equivalents, changes and modifications may be resorted to without departing from the spirit and scope of the invention. Therefore, it is understood that such equivalents are intended to be included herein.

0204922

## CLAIMS

1. An immunohistochemical assay for detecting p53 antigen in human cells or tissue specimens comprising:

    a) fixing human cells or tissue on a slide;

    b) contacting the cells or tissue with antibody to p53;

    c) contacting the cells or tissue with anti-Ig labeled either directly or indirectly with an enzyme;

    d) contacting the cells or tissue with a chromogenic indicator; and

    e) examining the slide for the presence of p53.

2. The immunohistochemical assay of claim 1 wherein the slide is pretreated with a positively-charged polymer solution and the cells or tissue are then treated with a fixative.

3. The immunohistochemical assay of claim 2 wherein the polymer solution is poly-1-lysine and the fixative is Zamboni's fixative.

4. The immunohistochemical assay of claim 1 wherein the anti-p53 antibody is a mouse anti-p53 monoclonal antibody.

5. The immunohistochemical assay of claim 4 wherein the anti-Ig is anti-mouse IgG, and the enzyme label is a mouse peroxidase, anti-peroxidase (PAP) complex.

6. An immunohistochemical assay for detection of p53 comprising:

a) treating a microscope slide with poly-1-lysine;

b) cutting a frozen section of tissue and transferring the tissue to the microscope slide;

c) fixing the frozen tissue on the slide in Zamboni's fixative;

d) contacting the tissue with a mouse anti-p53 monoclonal antibody;

e) contacting the tissue with an anti-mouse IgG antibody;

d) contacting the tissue with a mouse peroxidase, anti-peroxidase (PAP) complex;

e) contacting the tissue with a chromogen substrate solution; and

f) examining the slide microscopically for presence of stained cells.

7. The immunohistochemical assay of claim 6 wherein the chromogen substrate is a solution comprising diaminobenzidine and hydrogen peroxide.